# EUROPÄISCHE PATENTSCHRIFT

(11) **EP 1 551 816 B1**
(45) Veröffentlichungstag und Bekanntmachung des Hinweises auf die Patenterteilung: **14.04.2010**
(21) Anmeldenummer: 03779794.1
(22) Anmeldetag: 26.09.2003
(51) Int. Cl.: C07D 277/56, A01N 37/22, A01N 43/78

(54) **THIAZOLYLBIPHENYLAMIDE**
THIAZOLYL BIPHENYL AMIDES
AMIDES THIAZOLYLE BIPHENYLE

(30) Priorität: 09.10.2002 DE 10246959
(43) Veröffentlichungstag der Anmeldung: 13.07.2005
(73) Patentinhaber: Bayer CropScience AG, 40789 Monheim (DE)
(72) Erfinder: DUNKEL, Ralf, 69001 Lyon (FR); ELBE, Hans-Ludwig, 42329 Wuppertal (DE); RIECK, Heiko, 69110 ste. Foy les Lyon (FR); WACHENDORFF-NEUMANN, Ulrike, 56566 Neuwied (DE); KUCK, Karl-Heinz, 40764 Langenfeld (DE)
(86) Internationale Anmeldenummer: PCT/EP2003/010758
(87) Internationale Veröffentlichungsnummer: WO 2004/035555

(56) Entgegenhaltungen:
- EP-A- 0 545 099
- WO-A-2002/059086
- DE-A- 10 204 391

## Beschreibung

Die vorliegende Erfindung betrifft Thiazolylbiphenylamide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxanilide fungizide Eigenschaften besitzen (vergleiche z.B. EP 0 545 099). Die Wirksamkeit der dort beschriebenen Stoffe ist gut, lässt aber bei niedrigen Aufwandmengen in manchen Fällen zu wünschen übrig.

Es wurden nun Thiazolylbiphenylamide der Formel (I) 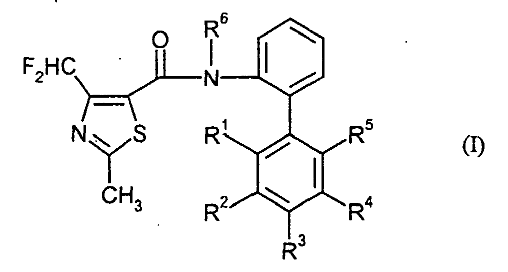 in welcher
- R¹, R², R³, R⁴ und R⁵: unabhängig voneinander für Wasserstoff, Halogen, Cyano, Nitro, C₁- C₆-Alkyl, C₂C₆-Alkenyl, C₁-C₄-Alkoxy, C₁-C₄-Alkylthio, C₁-C₄-Alkylsulfonyl, C₃- C₆-Cycloalkyl, oder für C₁-C₄-Halogenalkyl, C₁-C₄-Halogenalkoxy, C₁-C₄-Halogen- alkylthio oder C₁-C₄-Halogenalkylsulfonyl mit jeweils 1 bis 5 Halogenatomen stehen,
- R¹ und R² oder R² und R³: außerdem gemeinsam für gegebenenfalls durch Halogen oder C₁- C₆-Alkyl substituiertes Alkenylen stehen,
- R⁶: für C₁-C₈-Alkyl, C₁-C₆-Alkylsulfinyl, C₁-C₆-Alkylsulfonyl, C₁-C₄-Alkoxy-C₁-C₄- alkyl, C₃-C₈-Cycloalkyl; C₁-C₆-Halogenalkyl, C₁-C₄-Halogenalkylsulfanyl, C₁-C₄- Halogenalkylsulfinyl, C₁-C₄-Halogenalkylsulfonyl, Halogen-C₁-C₄-alkoxy-C₁-C₄-al- kyl, C₃-C₈-Halogencycloalkyl mit jeweils 1 bis 9 Fluor-, Chlor- und/oder Brom- atomen; -COR⁷, -CONR⁸R⁹ oder -CH₂NR¹⁰R¹¹ steht,
- R⁷: für Wasserstoff, C₁-C₈-Alkyl, C₁-C₈-Alkoxy, C₁-C₄-Alkoxy-C₁-C₄-alkyl, C₃-C₈- Cycloalkyl; C₁-C₆-Halogenalkyl, C₁-C₆-Halogenalkoxy, Halogen-C₁-C₄-alkoxy-C₁- C₄-alkyl, C₃-C₈-Halogencycloalkyl mit jeweils 1 bis 9 Fluor-, Chlor- und/oder Bromatomen oder 4-(Difluormethyl)-2-methyl-1,3-thiazol-2-yl steht,
- R⁸ und R⁹: unabhängig voneinander für Wasserstoff, C₁-C₈-Alkyl, C₁-C₄-Alkoxy-C₁-C₄-alkyl, C₃-C₈-Cycloalkyl, C₁-C₈-Halogenalk-yl, Halogen-C₁-C₄-alkoxy-C₁-C₄-alkyl, C₃-C₈- Hälogencycloalkyl mit jeweils 1 bis 9 Fluor-, Chlor- und/oder Bromatomen stehen,
- R⁸ und R⁹: außerdem gemeinsam mit dem Stickstoffatom, an das sie gebunden sind, einen ge- gebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
- R¹⁰ und R¹¹: unabhängig voneinander für Wasserstoff, C₁-C₈-Alkyl, C₃-C₈-Cycloalkyl; C₁-C₈- Halogenalkyl, C₃-C₈-Halogencycloalkyl mit jeweils 1 bis 9 Fluor-, Chlor- und/oder Bromatomen stehen,
- R¹⁰ und R¹¹: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
- R¹²: für Wasserstoff oder C₁-C₆-Alkyl steht.

### Weiterhin wurde gefunden, dass man Thiazolylbiphenylamide der Formel (I) erhält, indem man

### (A) Thiazolylbiphenylamide der Formel (II)

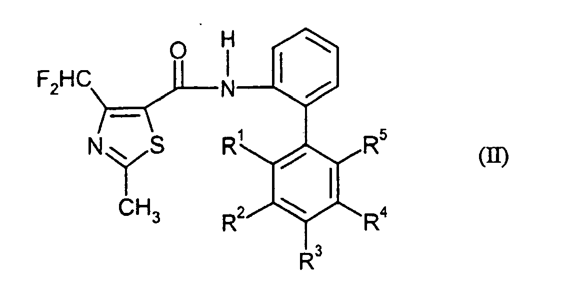

in welcher
- R¹, R², R³, R⁴ und R⁵: die oben angegebenen Bedeutungen haben,
mit einem Halogenid der Formel (III)

R⁶-X (III)

in welcher
- R⁶: die oben angegebenen Bedeutungen hat und
- X: für Chlor, Brom oder Iod steht,
in Gegenwart einer Base und in Gegenwart eines Verdünnungsmittels umsetzt.

Schließlich wurde gefunden, dass die Thiazolylbiphenylamide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Thiazolylbiphenylamide der Formel (I) eine wesentlich bessere fungizide Wirksamkeit als die konstitutionell ähnlichsten, vorbekannten Wirkstoffe gleicher Wirkungsrichtung.

Die erfindungsgemäßen Thiazolylbiphenylamide sind durch die Formel (I) allgemein definiert. Bevorzugte Definitionen der in den vorstehenden und nachstehenden Formeln genannten Substituenten sind im Folgenden angegeben. Sie gelten für die Vor- und Zwischenprodukte gleichermaßen.
- R¹, R², R³, R⁴ und R⁵: stehen unabhängig voneinander bevorzugt für Wasserstoff, Fluor, Chlor, Brom, Cyano, Nitro, Methyl, Ethyl, n- oder iso-Propyl, n-, iso-, sec- oder tert- Butyl, Methoxy, Ethoxy, Methylthio, Ethylthio, n- oder iso-Propylthio, Cyclopropyl, Trifluormethyl, Trichlormethyl, Trifluorethyl, Difluormethoxy, Trifluormethoxy, Difluorchlormethoxy, Trifluorethoxy, Difluormethylthio, Difluorchlormethylthio oder Trifluormethylthio.
- R¹ und R² oder R² und R³: stehen außerdem gemeinsam bevorzugt für gegebenenfalls durch Fluor, Chlor, Brom oder Methyl substituiertes Butadien-diyl.
- R⁶: steht bevorzugt für C₁-C₆-Alkyl, C₁-C₄-Alkylsulfinyl, C₁-C₄-Alkylsulfonyl, C₁-C₃- Alkoxy-C₁-C₃-alkyl, C₃-C₆-Cycloalkyl; C₁-C₄-Halogenalkyl, C₁-C₄-Halogenalkylsul- fanyl, C₁-C₄-Halogenalkylsulfinyl, C₁-C₄-Halogenalkylsulfonyl, Halogen-C₁-C₃- alkoxy-C₁-C₃-alkyl, C₃-C₆-Halogencycloalkyl mit jeweils 1 bis 9 Fluor-, Chlor- und/oder Bromatomen; -COR⁷, -CONR⁸R⁹ oder -CH₂NR¹⁰R¹¹.
- R⁷: steht bevorzugt für Wasserstoff, C₁-C₆-Alkyl, C₁-C₄-Alkoxy, C₁-C₃-Alkoxy-C₁-C₃- alkyl, C₃-C₆-Cycloalkyl; C₁-C₄-Halogenalkyl, C₁-C₄-Halogenalkoxy, Halogen-C₁-C₃- alkoxy-C₁-C₃-alkyl, C₃-C₆-Halogencycloalkyl mit jeweils 1 bis 9 Fluor-, Chlor- und/oder Bromatomen oder 4-(Difluormethyl)-2-methyl-1,3-thiazol-2-yl.
- R⁸ und R⁹: stehen unabhängig voneinander bevorzugt für Wasserstoff, C₁-C₆-Alkyl, C₁-C₃- Alkoxy-C₁-C₃-alkyl, C₃-C₆-Cycloalkyl; C₁-C₄-Halogenalkyl, Halogen-C₁-C₃-alkoxy- C₁-C₃-alkyl, C₃-C₆-Halogencycloalkyl mit jeweils 1 bis 9 Fluor-, Chlor- und/oder Bromatomen.
- R⁸ und R⁹: bilden außerdem gemeinsam mit dem Stickstoffatom, an das sie gebunden sind, bevorzugt einen gegebenenfalls einfach bis vierfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Hetero- atome aus der Reihe Sauerstoff, Schwefel oder NR¹² enthalten kann.
- R¹⁰ und R¹¹: stehen unabhängig voneinander bevorzugt für Wasserstoff, C₁-C₆-Alkyl, C₃-C₆- Cycloalkyl; C₁-C₄-Halogenalkyl, C₃-C₆-Halogencycloalkyl mit jeweils 1 bis 9 Fluor-, Chlor- und/oder Bromatomen.
- R¹⁰ und R¹¹: bilden außerdem gemeinsam mit dem Stickstoffatom, an das sie gebunden sind, bevorzugt einen gegebenenfalls einfach oder mehrfach, gleich oder verschieden durch Halogen oder C₁-C₄-Alkyl substituierten gesättigten Heterocyclus mit 5 bis 8 Ringatomen, wobei der Heterocyclus 1 oder 2 weitere, nicht benachbarte Heteroatome aus der Reihe Sauerstoff, Schwefel oder NR¹² enthalten kann.
- R¹²: steht bevorzugt für Wasserstoff oder C₁-C₄-Alkyl.
- R¹, R², R³, R⁴ und R⁵: stehen unabhängig voneinander besonders bevorzugt für Wasserstoff, Fluor, Chlor, Brom, Cyano, Methyl, Methoxy, Methylthio, Trifluormethyl, Difluor- methoxy, Trifluormethoxy, Difluormethylthio oder Trifluormethylthio.
- R⁶: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Pro- pylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylsulfanyl, Difluorchlor- methylsulfanyl, Trifluormethylsulfanyl, Trifluormethylsulfinyl, Trifluormethyl- sulfonyl, Trifluormethoxymethyl; -COR⁷, -CONR⁸R⁹ oder -CH₂NR¹⁰R¹¹.
- R⁷: steht besonders bevorzugt für Wasserstoff, Methyl, Ethyl, n- oder iso-Propyl, tert- Butyl, Methoxy, Ethoxy, tert-Butoxy, Cyclopropyl; Trifluormethyl, Trifluormethoxy oder 4-(Difluormethyl)-2-methyl-1,3-thiazol-2-yl.
- R⁸ und R⁹: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁸ und R⁹: bilden außerdem gemeinsam mit dem Stickstoffatom, an das sie gebunden sind, besonders bevorzugt einen gegebenenfalls einfach bis vierfach, gleich oder verschie- den durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹² substituiert sein kann.
- R¹⁰ und R¹¹: stehen unabhängig voneinander besonders bevorzugt für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R¹⁰ und R¹¹: bilden außerdem gemeinsam mit dem Stickstoffatom, an das sie gebunden sind, besonders bevorzugt einen gegebenenfalls einfach bis vierfach, gleich oder verschie- den durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹² substituiert sein kann.
- R¹²: steht besonders bevorzugt für Wasserstoff, Methyl, Ethyl, n- oder iso-Propyl, n-, iso- , sec- oder tert-Butyl.

Ganz besonders bevorzugt sind Thiazolylbiphenylamide der Formel (I), in welcher jeweils vier der Reste R¹, R², R³, R⁴ und R⁵ für Wasserstoff stehen.

Ganz besonders bevorzugt sind Thiazolylbiphenylamide der Formel (I), in welcher
- R¹, R², R⁴ und R⁵: jeweils für Wasserstoff stehen und
- R³: die oben angegebenen Bedeutungen hat.

Ganz besonders bevorzugt sind Thiazolylbiphenylamide der Formel (I), in welcher
- R¹, R², R⁴ und R⁵: jeweils für Wasserstoff stehen und
- R³: für Fluor, Chlor, Brom, Methyl, Trifluormethyl, Trifluormethoxy oder Trifluor- methylthio steht.

Ganz besonders bevorzugt sind Thiazolylbiphenylamide der Formel (I), in welcher
- R², R⁴ und R⁵: für jeweils Wasserstoff stehen und
- R¹ und R³: unabhängig voneinander die oben angegebenen Bedeutungen haben.

Ganz besonders bevorzugt sind Thiazolylbiphenylamide der Formel (I), in welcher
- R², R⁴ und R⁵: für jeweils Wasserstoff stehen und
- R¹ und R³: unabhängig voneinander für Fluor, Chlor, Brom, Methyl oder Trifluormethylstehen.

Ganz besonders bevorzugt sind Thiazolylbiphenylamide der Formel (I), in welcher
- R¹, R⁴ und R⁵: jeweils für Wasserstoff stehen und
- R² und R³: unabhängig voneinander die oben angegebenen Bedeutungen haben.

Ganz besonders bevorzugt sind Thiazolylbiphenylamide der Formel (I), in welcher
- R¹, R⁴ und R⁵: jeweils für Wasserstoff stehen und
- R² und R³: unabhängig voneinander für Fluor, Chlor, Brom, Methyl oder Trifluormethyl stehen.

Ganz besonders bevorzugt sind Thiazolylbiphenylamide der Formel (I), in welcher
- R¹, R³ und R⁵: jeweils für Wasserstoff stehen und
- R² und R⁴: unabhängig voneinander die oben angegebenen Bedeutungen haben.

Ganz besonders bevorzugt sind Thiazolylbiphenylamide der Formel (I), in welcher
- R¹, R³ und R⁵: jeweils für Wasserstoff stehen und
- R² und R⁴: unabhängig voneinander für Fluor, Chlor, Brom, Methyl oder Trifluormethylstehen.

Ganz besonders bevorzugt sind Thiazolylbiphenylamide der Formel (I), in welcher
- R⁶: für -COR⁷ steht und R⁷ für 4-(Difluormethyl)-2-methyl-1,3-thiazol-2-yl steht.

Ganz besonders bevorzugt sind Thiazolylbiphenylamide der Formel (I), in welcher
- R⁶: für -COR⁷ steht und R⁷ für Methyl, Ethyl, Cyclopropyl oder Trifluormethyl, insbesondere Methyl steht.

Ganz besonders bevorzugt sind Thiazolylbiphenylamide der Formel (I), in welcher
- R⁶: für -CHO steht.

Ganz besonders bevorzugt sind Thiazolylbiphenylamide der Formel (I), in welcher
- R⁶: für Methyl, Ethyl, n- oder iso-Propyl, n-, iso-, sec- oder tert-Butyl, Methylsulfinyl, Methylsulfonyl, Methoxymethyl, Ethoxyethyl, Cyclopropyl, Cyclopentyl, Cyclohexyl, Trifluormethyl, Trichlormethyl, Trifluormethylsulfanyl, Trifluorme- thylsulfinyl, Trifluormethylsulfonyl, Trifluormethoxymethyl, insbesondere Methyl, iso-Propyl oder Cyclopropyl steht.

Eine bevorzugte Gruppe sind Thiazolylbiphenylamide der Formel (I-a) 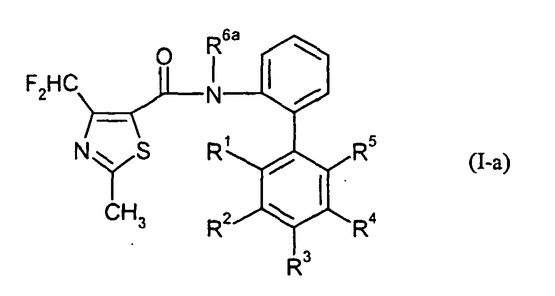 in welcher
- R¹, R², R³, R⁴ und R⁵: unabhängig voneinander für Wasserstoff, Halogen, Cyano, Nitro, C₁- C₆-Alkyl, C₂-C₆-Alkenyl, C₁-C₄-Alkoxy, C₁-C₄-Alkylthio, C₁-C₄-Alkylsulfonyl, C₃- C₆-Cycloalkyl, oder für C₁-C₄-Halogenalkyl, C₁-C₄-Halogenalkoxy, C₁-C₄- Halogenalkylthio oder C₁-C₄-Halogenalkylsulfonyl mit jeweils 1 bis 5 Halogen- atomen stehen,
- R¹ und R² oder R² und R³: außerdem gemeinsam für gegebenenfalls durch Halogen oder C₁- C₆-Alkyl substituiertes Alkenylen stehen,
- R^{6a}: für C₁-C₈-Alkyl, C₁-C₆-Alkylsulfinyl, C₁-C₆-Alkylsulfonyl, C₁-C₄-Alkoxy-C₁-C₄- alkyl, C₃-C₈-Cycloalkyl; C₁-C₆-Halogenalkyl, C₁-C₄-Halogenalkylsulfanyl, C₁-C₄- Halogenalkylsulfinyl, C₁-C₄-Halogenalkylsulfonyl, Halogen-C₁-C₄-alkoxy-C₁-C₄- alkyl, : C₃-C₈-Halogencycloalkyl mit jeweils 1 bis 9 Fluor-, Chlor- und/oder Bromatomen; -COR^{7a}, -CONR⁸R⁹ oder -CH₂NR¹⁰R¹¹ steht,
- R^{7a}: für Wasserstoff, C₃-C₈-Cycloalkyl; C₃-C₈-Halogencycloalkyl mit jeweils 1 bis 9 Fluor-, Chlor- und/oder Bromatomen oder 4-(Difluormethyl)-2-methyl-1,3-thiazol-2- yl steht,
- R⁸ und R⁹: unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈- Halogencycloalkyl mit jeweils 1 bis 9 Fluor-, Chlor- und/oder Bromatomen stehen,
- R⁸ und R⁹: außerdem gemeinsam mit dem Stickstoffatom, an das sie gebunden sind, einen ge- gebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
- R¹⁰ und R¹¹: unabhängig voneinander für Wasserstoff, C₁-C₈-Alkyl, C₃-C₈-Cycloalkyl; C₁-C₈- Halogenalkyl, C₃-C₈-Halogencycloalkyl mit jeweils 1 bis 9 Fluor-, Chlor- und/oder Bromatomen stehen,
- R¹⁰ und R¹¹: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
- R¹²: für Wasserstoff oder C₁-C₆-Alkyl steht.

Die erfindungsgemäßen Thiazolylbiphenylamide sind durch die Formel (I-a) allgemein definiert. Bevorzugte Definitionen der in dieser Formel genannten Substituenten sind im Folgenden angegeben.

R¹, R², R³, R⁴, R⁵, R⁸, R⁹, R¹⁰, R¹¹ und R¹² stehen unabhängig voneinander bevorzugt, besonders bevorzugt bzw. ganz besonders bevorzugt für diejenigen Bedeutungen die bereits im Zusammenhang mit der Beschreibung der erfindungsgemäßen Stoffe der Formel (I) als bevorzugt, besonders bevorzugt bzw. ganz besonders bevorzugt für diese Reste angegeben wurden.
- R^{6a}: steht bevorzugt für C₁-C₆-Alkyl, C₁-C₄-Alkylsulfinyl, C₁-C₄-Alkylsulfonyl, C₁-C₃- Alkoxy-C₁-C₃-alkyl, C₃-C₆-Cycloalkyl; C₁-C₄-Halogenalkyl, C₁-C₄-Halogenalkylsul- fanyl, C₁-C₄-Halogenalkylsulfinyl, C₁-C₄-Halogenalkylsulfonyl, Halogen-C₁-C₃- alkoxy-C₁-C₃-alkyl, C₃-C₆-Halogencycloalkyl mit jeweils 1 bis 9 Fluor-, Chlor- und/oder Bromatomen; -COR^{7a}, -CONR⁸R⁹ oder -CH₂NR¹⁰R¹¹.
- R^{7a}: steht bevorzugt für Wasserstoff, C₃-C₆-Cycloalkyl; C₃-C₆-Halogencycloalkyl mit jeweils 1 bis 9 Fluor-, Chlor- und/oder Bromatomen oder 4-(Difluormethyl)-2- methyl-1,3-thiazol-2-yl.
- R^{6a}: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Pro- pylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylsulfanyl, Difluorchlor- methylsulfanyl, Trifluormethylsulfanyl, Trifluormethylsulfinyl, Trifluormethyl- sulfonyl, Trifluormethoxymethyl; -COR^{7a}, -CONR⁸R⁹ oder -CH₂NR¹⁰R¹¹.
- R⁷: steht besonders bevorzugt für Wasserstoff, Cyclopropyl oder 4-(Difluormethyl)-2- methyl-1,3-thiazol-2-yl.

Weiterhin bevorzugt sind Thiazolylbiphenylamide der Formel (I-a), in welcher R¹, R², R³, R⁴ und R⁵ nicht gleichzeitig für Wasserstoff stehen.

Weiterhin bevorzugt sind Thiazolylbiphenylamide der Formel (I-a), in welcher R¹, R², R³, R⁴ und R⁵ die oben angegebenen Bedeutungen haben, aber nicht für Halogen stehen.

Weiterhin bevorzugt sind Thiazolylbiphenylamide der Formel (I-a), in welcher R¹, R², R³, R⁴ und R⁵ nicht gleichzeitig für Wasserstoff stehen und außerdem unabhängig voneinander nicht für Halogen stehen.

Verwendet man *N*-(4'-Brom-1,1'-biphenyl-2-yl)-4-(difluormethyl)-2-methyl-1,3-thiazol-5-carboxamid und Acetylchlordi als Ausgangsstoffe, so kann der Verlauf des erfindungsgemäßen Verfahrens (A) durch folgende Reaktionsgleichung veranschaulicht werden:

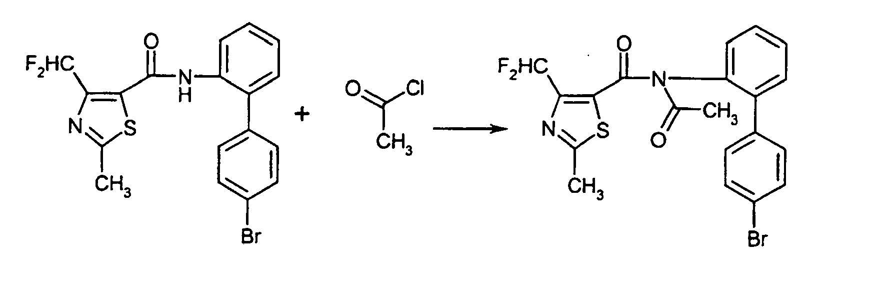

Die zur Durchführung des erfindungsgemäßen Verfahrens (A) als Ausgangsstoffe benötigten Thiazolylbiphenylamide sind durch die Formel (II) allgemein definiert. In dieser Formel stehen R¹, R², R³, R⁴ und R⁵ bevorzugt, besonders bevorzugt bzw. ganz besonders bevorzugt für diejenigen Bedeutungen, die bereits in Zusammenhang mit der Beschreibung der erfindungsgemäßen Stoffe der Formel (I) für diese Reste als bevorzugt, besonders bevorzugt bzw. ganz besonders bevorzugt angegeben wurden.

Thiazolylbiphenylamide der Formel (II) sind in DE-A 102 04 391 beschrieben. Sie lassen sich herstellen, indem man
a) Difluormethylthiazolylcarbonsäurehalogenide der Formel (IV) 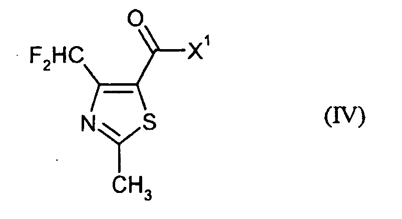 in welcher
   - X¹: für Halogen steht,
   mit Anilinderivaten der Formel (V) 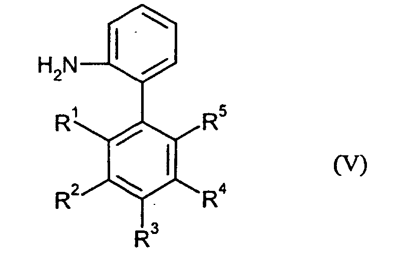 in welcher
   - R¹, R², R³, R⁴ und R⁵: die oben angegebenen Bedeutungen haben,
   gegebenenfalls in Gegenwart eines Säurebindemittels (z.B. Triethylamin) und gegebenenfalls in Gegenwart eines Verdünnungsmittels (z.B. Tetrahydrofuran) umsetzt, oder
b) Difluormethylthiazolylcarboxhalogenanilide der Formel (VI) 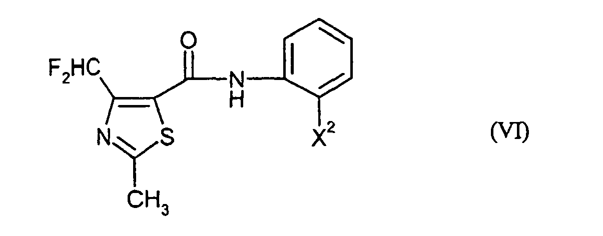 in welcher
   - X²: für Brom oder Iod steht,
   mit Boronsäurederivaten der Formel (VII) 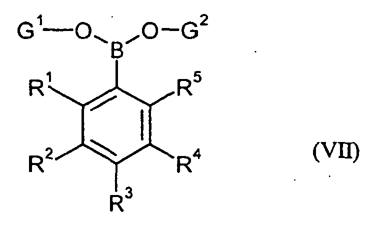 in welcher
   - R¹, R², R³, R⁴ und R⁵: die oben angegebenen Bedeutungen haben,
   - G¹ und G²: jeweils für Wasserstoff oder zusammen für Tetramethylethylen stehen,
   in Gegenwart eines Katalysators (z.B. 1,1'-Bis(diphenylphosphino)ferrocenpalladium(II)chlorid), gegebenenfalls in Gegenwart eines Säurebindemittels (z.B. Kaliumacetat) und gegebenenfalls in Gegenwart eines Verdünnungsmittels (z.B. Dimethylsulfoxid) umsetzt, oder
c) Thiazolylbiphenylamid-Boronsäure-Derivate der Formel (VIII) 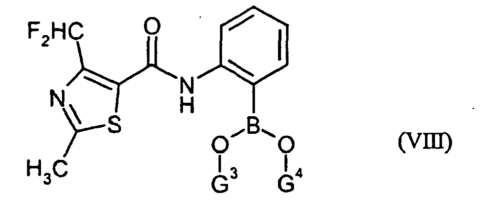 in welcher
   - G³ und G⁴: jeweils für Wasserstoff oder zusammen für Tetramethylethylen stehen,
   mit Halogenbenzolderivaten der Formel (IX) 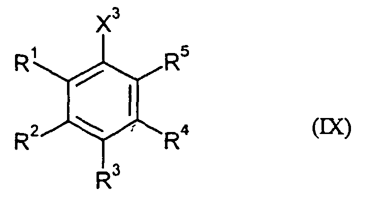 in welcher
   - R¹, R², R³, R⁴ und R⁵: die oben angegebenen Bedeutungen haben,
   - X³: für Brom, Iod oder Trifluormethylsulfonyloxy steht,
   in Gegenwart eines Katalysators (z.B. 1,1'-Bis(diphenylphosphino)ferrocenpalladium(II)chlorid), gegebenenfalls in Gegenwart eines Säurebindemittels (z.B. Kaliumacetat) und gegebenenfalls in Gegenwart eines Verdünnungsmittels (z.B. Dimethylsulfoxid) umsetzt.
   Die zur Durchführung des erfindungsgemäßen Verfahrens a) als Ausgangsstoffe benötigten Difluormethylthiazolylcarbonsäurehalogenide sind durch die Formel (IV) allgemein definiert. In dieser Formel (IV) steht X¹ bevorzugt für Chlor.
   Die Difluormethylthiazolylcarbonsäurehalogenide der Formel (IV) sind bekannt und/ oder lassen sich nach bekannten Verfahren herstellen (vergleiche z.B. EP 0 276 177).
   Die weiterhin zur Durchführung des erfindungsgemäßen Verfahrens a) als Ausgangsstoffe benötigten Aniline sind durch die Formel (V) allgemein definiert. In dieser Formel (V) stehen R¹, R², R³, R⁴ und R⁵ bevorzugt, besonders bevorzugt bzw. ganz besonders bevorzugt für diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.
   Die Anilin-Derivate der Formel (V) sind bekannt und/oder lassen sich nach bekannten Methoden herstellen (vgl. z.B. Bull. Korean Chem. Soc. 2000, 21, 165-166; Chem. Pharm. Bull. 1992, 40, 240-4; JP 09132567).
   Die zur Durchführung des erfindungsgemäßen Verfahrens b) als Ausgangsstoffe benötigten Difluormethylthiazolylcarboxhalogenanilide sind durch die Formel (VI) allgemein definiert. In dieser Formel (VI) steht X² bevorzugt für Brom oder Iod.
   Die Difluormethylthiazolylcarboxhalogenanilide der Formel (VI) lassen sich herstellen, indem man
d) Difluormethylthiazolylcarbonsäurehalogenide der Formel (IV) 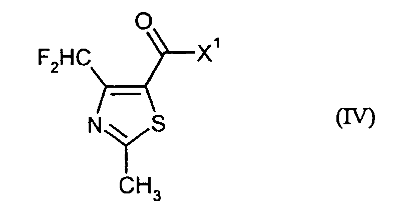 in welcher
   - X¹: für Halogen steht,
   mit 2-Bromanilin oder 2-Iodanilin umsetzt.
   Die zur Durchführung des erfindungsgemäßen Verfahrens d) als Ausgangsstoffe benötigten Difluormethylthiazolylcarbonsäurehalogenide der Formel (IV) sind bereits weiter oben im Zusammenhang mit dem erfindungsgemäßen Verfahren a) beschrieben worden.
   Die weiterhin zur Durchführung des erfindungsgemäßen Verfahrens d) als Ausgangsstoffe benötigten Stoffe 2-Bromanilin oder 2-Iodanilin sind bekannte Synthesechemikalien.
   Die weiterhin zur Durchführung des erfindungsgemäßen Verfahrens b) als Ausgangsstoffe benötigten Boronsäurederivate sind durch die Formel (VII) allgemein definiert. In dieser Formel (VII) stehen R¹, R², R³, R⁴ und R⁵ bevorzugt, besonders bevorzugt bzw. ganz besonders bevorzugt für diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. G¹ und G² stehen bevorzugt jeweils für Wasserstoff oder zusammen für Tetramethylethylen.
   Boronsäurederivate der Formel (VII) sind bekannte Synthesechemikalien. Sie können auch unmittelbar vor der Reaktion direkt aus Halogenbenzolderivaten und Boronsäureestern hergestellt und ohne Aufarbeitung weiter umgesetzt werden (siehe auch die Herstellungsbeispiele).
   Die zur Durchführung des erfindungsgemäßen Verfahrens c) als Ausgangsstoffe benötigten Thiazolylbiphenylamid-Boronsäure-Derivate sind durch die Formel (VIII) allgemein definiert. In dieser Formel (VIII) stehen G³ und G⁴ bevorzugt jeweils für Wasserstoff oder zusammen für Tetramethylethylen.
   Die Thiazolylbiphenylamid-Boronsäure-Derivate der Formel (VIII) lassen sich herstellen, indem man
e) Difluormethylthiazolylcarbonsäurehalogenide der Formel (IV) 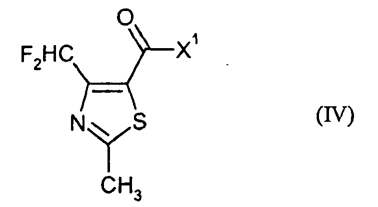 in welcher
   - X¹: für Halogen steht,
   mit Anilinboronsäurederivaten der Formel (X) 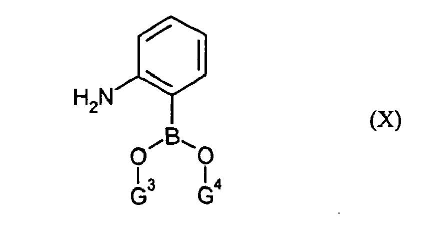 in welcher
   - G³ und G⁴: die oben angegebenen Bedeutungen haben,
   gegebenenfalls in Gegenwart eines Säurebindemittels und gegebenenfalls in Gegenwart eines Verdünnungsmittels umsetzt.

Die zur Durchführung des erfindungsgemäßen Verfahrens e) als Ausgangsstoffe benötigten Difluormethylthiazolylcarbonsäurehalogenide der Formel (IV) sind bereits weiter oben im Zusammenhang mit dem erfindungsgemäßen Verfahren a) beschrieben worden.

Die weiterhin zur Durchführung des erfindungsgemäßen Verfahrens e) als Ausgangsstoffe benötigten Anilinboronsäurederivate sind durch die Formel (X) allgemein definiert. In dieser Formel (X) stehen G³ und G⁴ bevorzugt jeweils für Wasserstoff oder zusammen für Tetramethylethylen.

Die zur Durchführung des erfindungsgemäßen Verfahrens e) als Ausgangsstoffe benötigten Anilinboronsäurederivate der Formel (X) sind bekannte Synthesechemikalien.

Die weiterhin zur Durchführung des erfindungsgemäßen Verfahrens c) als Ausgangsstoffe benötigten Halogenbenzolderivate sind durch die Formel (IX) allgemein definiert. In dieser Formel (IX) stehen R¹, R², R³, R⁴ und R⁵ bevorzugt, besonders bevorzugt bzw. ganz besonders bevorzugt für diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. X³ steht bevorzugt für Brom, Iod oder Trifluormethylsulfonyloxy.

Die außerdem zur Durchführung des erfindungsgemäßen Verfahrens (A) als Ausgangsstoffe benötigten Halogenide sind durch die Formel (III) allgemein definiert. In dieser Formel steht R⁶ bevorzugt, besonders bevorzugt bzw. ganz besonders bevorzugt für diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. X steht bevorzugt für Chlor oder Brom.

Halogenide der Formel (III) sind bekannte Synthesechemikalien.

Als Verdünnungsmittel zur Durchführung des erfindungsgemäßen Verfahrens (A) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzoyl, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (A) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (A) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (A) zur Herstellung der Verbindungen der Formel (I) setzt man pro Mol des Thiazolylbiphenylamids der Formel (II) im allgemeinen 0,2 bis 5 Mol, vorzugsweise 0,5 bis 2 Mol an Halogenid der Formel (III) ein.

Als Verdünnungsmittel zur Durchführung der erfindungsgemäßen Verfahren a), d) und e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Die erfindungsgemäßen Verfahren a), d) und e) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren a), d) und e) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens a) zur Herstellung der Verbindungen der Formel (II) setzt man pro Mol des Difluormethylthiazolylcarbonsäurehalogenides der Formel (IV) im allgemeinen 0,2 bis 5 Mol, vorzugsweise 0,5 bis 2 Mol an Anilinderivat der Formel (V) ein.

Zur Durchführung des erfindungsgemäßen Verfahrens d) zur Herstellung der Verbindungen der Formel (V) setzt man pro Mol des Difluormethylthiazolylcarbonsäurehalogenides der Formel (IV) im allgemeinen 0,2 bis 5 Mol, vorzugsweise 0,5 bis 2 Mol an 2-Bromanilin oder 2-Iodanilin ein.

Zur Durchführung des erfindungsgemäßen Verfahrens e) zur Herstellung der Verbindungen der Formel (VIII) setzt man pro Mol des Difluormethylthiazolylcarbonsäurehalogenides der Formel (IV) im allgemeinen 0,2 bis 5 Mol, vorzugsweise 0,5 bis 2 Mol an Anilinboronsäurederivat der Formel (X) ein.

Als Verdünnungsmittel zur Durchführung der erfindungsgemäßen Verfahren b) und c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahrens b) und c) in einem größeren Bereich variiert werden. Im allgemeinen arbeitet man bei Temperaturen von 0°C bis 150°C, vorzugsweise bei Temperaturen von 20°C bis 110°C.

Die erfindungsgemäßen Verfahren b) und c) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, fluoride, phosphate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumphosphat, Kaliumphosphat, Kaliumfluaorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b) und c) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-Palladium, Bis-(triphenylphosphin)-Palladiumdichlorid oder (1,1'-Bis(diphenylphosphino)ferrocenpalladium(II)chlorid) infrage.

Es kann auch ein Palladiumkomplex in der Reaktionsmischung erzeugt werden, wenn man ein Palladiumsalz und ein Komplexligand, wie beispielsweise Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-Tolyl)-phosphan, Natrium-3-(diphenylphosphino)benzolsulfonat, Tris-2-(Methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens b) zur Herstellung der Verbindungen der Formel (II) setzt man pro Mol des Difluormethylthiazolylcarboxhalogenanilide der Formel (VI) im allgemeinen 1 bis 15 Mol, vorzugsweise 2 bis 8 Mol an Boronsäurederivat der Formel (VII) ein.

Zur Durchführung des erfindungsgemäßen Verfahrens c) zur Herstellung der Verbindungen der Formel (II) setzt man pro Mol des Thiazolylbiphenylamid-Boronsäure-Derivates der Formel (VIII) im allgemeinen 1 bis 15 Mol, vorzugsweise 2 bis 8 Mol an Halogenbenzolderivat der Formel (IX) ein.

Die erfindungsgemäßen Verfahren (A), a), b), c) und d) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau einsetzen, wie beispielsweise gegen Venturia-, Botrytis-, Sclerotinia-, Rhizoctonia-, Uncinula-, Sphaerotheca-, Podosphaera-, Alternaria- und Colletotrichum-Arten. Mit gutem Erfolg werden auch Reiskrankheiten, wie Pyricularia- und Pellicularia-Arten, bekämpft.

Die erfindungsgemäßen Wirkstoffe eignen sich auch zur Steigerung des Emteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen. Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin;
Benalaxyl; Benodanil; Benomyl; Benthiavalicarb-isopropyl; Benzamacril; Benzamacrilisobutyl; Bilanafos; Binapacryl; Biphenyl; Bitertanol; Blasticidin-S; Bromuconazole; Bupirimate; Buthiobate; Butylamine;
Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram;
Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon;
Edifenphos; Epoxiconazole; Ethaboxam; Ethirimol; Etridiazole;
Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox;
Guazatine; Hexachlorobenzene; Hexaconazole; Hymexazol;
Imazalil; Imibenconazole; Iminoctadine triacetate; Iminoctadine tris(albesil; Iodocarb; Ipconazote; Iprobenfos; Iprodione; Iprovalicarb; Irumamycin; Isoprothiolane; Isovaledione; Kasugamycin; Kresoxim-methyl;
Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin;
Natamycin; Nicobifen; Nitrothal-isopropyl; Noviflumuron; Nuarimol;
Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur;
Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole;
Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamid; 3,4,5-Trichlor-2,6-pyridindicarbonitril; Actinovate; cis-1-(4-Chlorphenyl)-2-(1H-1,2,4-triazol-1-yl)-cycloheptanol; Methyl-1-(2,3-dihydro-2,2--dimethyl-1H-inden-1-yl)-1H-imidazol-5-carboxylat; Monokaliumcarbonat; N-(6-Methoxy-3-pyridinyl)-cyclopropancarboxamid; N-Butyl-8-(1,1-dimethylethyl)-1-oxaspiro[4.5]decan-3-amin; Natriumtetrathiocarbonat;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin IR-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
Eflusilanate, Emamectin, Emamectin-benzoate, Empenthrin (IR-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure, Kadethrin, Kempolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, OK-5101, OK-5201, OK-9601, OK-9602, OK-9701, OK-9802, Omethoate, Oxamyl, Oxydemeton-methyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphosmethyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos, Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
Vamidothion, Vaniliprole, Verbutin, Verticillium lecanii,
WL-108477, WL-40027, YI-5201, YI-5301, YI-5302, XMC, Xylylcarb, ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semicochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die zum Schutz technischer Materialien verwendeten Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

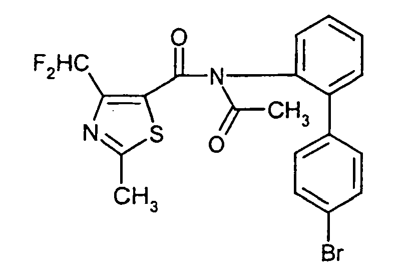

N-(4'-Brom-1,1'-biphenyl-2-yl)-4-(difluormethyl)-2-methyl-1,3-thiazol-5-carboxamid (0,3 g, 0,7 mmol) wird in Tetrahydrofuran (20 ml) vorleget und mit Natriumhydrid (60%ig, 34 mg, 0,85 mmol) versetzt. Nach 15 min bei Raumtemperatur wird Acetylchlorid (50 µl, 0,7 mmol) zugeben und 5 h bei 50°C gerührt.

Zur Aufarbeitung wird mit gesättigter Natriumhydrogencarbonat-Lösung gewaschen, mit Essigsäureethylester extrahiert, mit Natriumsulfat getrocknet und im Vakuum aufkonzentriert.

Man erhält 0,31g (95 % der Theorie) N-Acetyl-N-(4'-brom-1,1'-biphenyl-2-yl)-4-(difluormethyl)-2-methyl-1,3-thiazol-5-carboxamid mit dem logP (pH 2,3) = 3,61.

### Beispiel 2

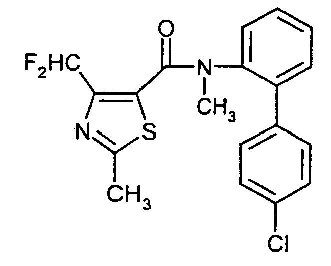

N-(4'-Chlor-1,1'-biphenyl-2-yl)-4-(difluormethyl)-2-methyl-1,3-thiazol-5-carboxamid (0,3 g, 0,8 mmol) wird in Tetrahydrofuran (20 ml) vorlegt und mit Natriumhydrid (60%ig, 23 mg, 1,0 mmol) versetzt. Nach 15 min bei Raumtemperatur wird Methyljodid (100 µl, 1,6 mmol) zugegeben und für 16 h refluxiert.

Zur Aufarbeitung wird mit Natriumhydrogencarbonat-Lösung gewaschen, mit Essigsäureethylester extrahiert, die organische Phase über Natriumsulfat getrocknet, filtriert und ein**geengt.**

Man erhält 0,25 g (80 % der Theorie) an *N*-(4'-Chlor-1,1'-biphenyl-2-yl)-4-difluormethyl)-N,2-dimethyl-1,3-thiazol-5-carboxamid mit dem logP (pH 2,3) = 3,34.

Analog den Beispielen 1 und 2, sowie entsprechend den Angaben in der allgemeinen Beschreibung des Verfahrens(A), werden die in der nachstehenden Tabelle 1 genannten Verbindungen der Formel (I) erhalten.

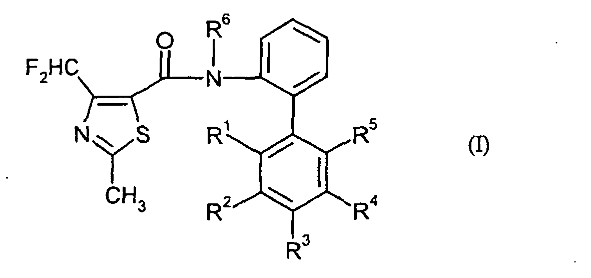

**Tabelle 1**

| **Bsp.** | **R¹** | **R²** | **R³** | **R⁴** | **R⁵** | **R⁶** | **logP** |
|---|---|---|---|---|---|---|---|
| 3 | H | CH₃ | Cl | H | H | 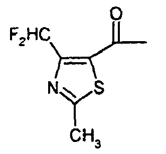 | 4,24 |
| 4 | H | Cl | H | Cl | H | 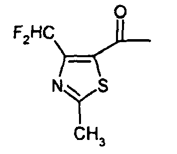 | 4,28 |
| 5 | H | H | Cl | H | H | 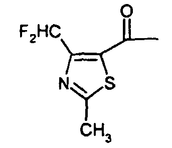 | 3,94 |
| 6 | H | H | Br | H | H | -CH₃ | 3,44 |
| 7 | H | H | F | H | H | -CH₃ | 2,99 |
| 8 | H | H | F | H | H | -COCH₃ | 3,16 |
| 9 | H | H | Cl | H | H | -COCH₂OCH₃ | 3,34 |

### Herstellung von Ausgangsstoffen der Formel (II)

### Beispiel (II-1)

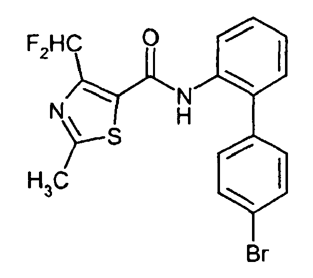

23,2 g (0,09 mol) 4'-Brom-1,1'-biphenyl-2-amin und 26,0 ml (0,19 mol) Triethylamin in 1,01 Tetrahydrofuran werden bei einer Temperatur zwischen -10 und -20 °C langsam mit einer Lösung von 21,8 g (0,10 mol) 2-Methyl-4-(difluormethyl)-1,3-thiazol-5-carbonylchlorid in 200 ml Tetrahydrofuran versetzt. Die Reaktionslösung wird für 2 h bei 0°C gerührt. Zur Aufarbeitung wird aufkonzentriert und mit Cyclohexan/Essigsäureethylester an Kieselgel chromatographiert.

Man erhält 27,8 g (70 % d. Th.) an *N*-(4'-Brom-1,1'-biphenyl-2-yl)-2-methyl-4-(difluormethyl)-1,3-thiazol-5-carboxamid mit dem logP (pH2,3) = 3,34 und dem Schmelzpunkt 151°C.

### Beispiel (II-2)

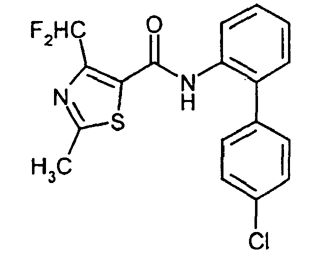

65,1 g (0,32 mol) 4'-Brom-1,1'-biphenyl-2-amin und 74,0 ml (0,53 mol) Triethylamin in 2,01 Tetrahydrofuran werden bei einer Temperatur zwischen -10°C und -20 °C langsam mit einer Lösung von 56,3 g (0,27 mol) 2-Methyl-4-(difluormethyl)-1,3-thiazol-5-carbonylchlorid in 500 ml Tetrahydrofuran versetzt. Die Reaktionslösung wird für 2 h bei 0°C gerührt. Zur Aufarbeitung wird aufkonzentriert und mit Cyclohexan/Essigsäureethylester an Kieselgel chromatographiert.

Man erhält 43,88 g (44,5 % d. Th.) an N-(4'-Chlor-1,1'-biphenyl-2-yl)-2-methyl-4-(difluormethyl)-1,3-thiazol-5-carboxamid mit dem logP (pH2,3) = 3,26 und dem Schmelzpunkt 144°C.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

| **Sphaerotheca-Test (Gurke) / protektiv** | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Sprühbelages werden die Pflanzen mit einer wässrigen Sporensuspension von *Sphaerotheca fuliginea* inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

| Tabelle A: **Sphaerotheca-Test (Gurke) / protektiv** | | | |
|---|---|---|---|
| Bsp. | Wirkstoff | Aufwandmenge an Wirkstoff in g/ha | % Wirkungsgrad |
| 4 | 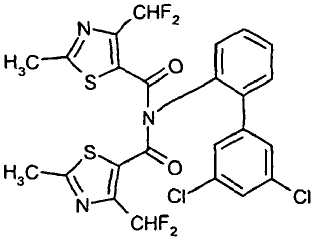 | 100 | 100 |
| 6 | 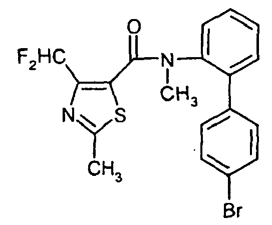 | 100 | 100 |
| 1 | 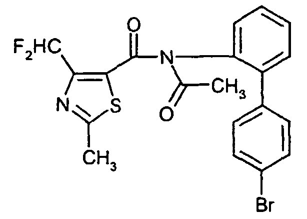 | 100 | 100 |
| 2 | 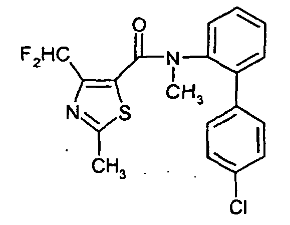 | 100 | 95 |

### Beispiel B

| **Venturia - Test (Apfel) / protektiv** | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

| Tabelle B: | | | |
|---|---|---|---|
| **Venturia-Test (Apfel) / protektiv** | | | |
| Bsp. | Wirkstoff | Aufwandmenge an Wirkstoff in g/ha | % Wirkungsgrad |
| 4 | 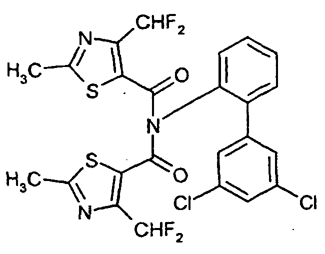 | 100 | 99 |
| 1 | 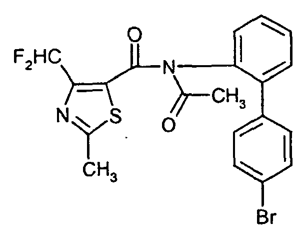 | 100 | 100 |

### Beispiel C

| **Botrytis-Test (Bohne) / protektiv** | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

| Tabelle C: | | | |
|---|---|---|---|
| **Botrytis-Test (Bohne) / protektiv** | | | |
| Bsp. | Wirkstoff | Aufwandmenge an Wirkstoff in g/ha | % Wirkungsgrad |
| 4 | 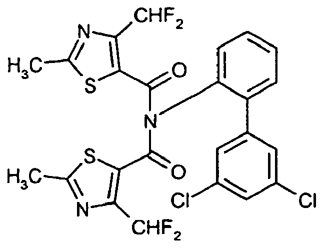 | 500 | 94 |
| 1 | 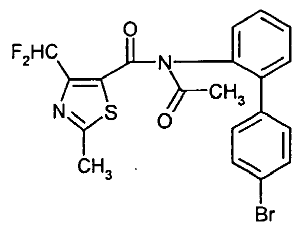 | 500 | 98 |

### Beispiel D

| **Alternaria-Test (Tomate) / protektiv** | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer wässrigen Sporensuspension von *Alternaria solani* inokuliert und stehen dann 24 Stunden bei 100 % relativer Feuchte. Anschließend stehen die Pflanzen bei ca. 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor.

| Tabelle D: | | | |
|---|---|---|---|
| **Alternaria-Test (Tomate) / protektiv** | | | |
| Bsp. | Wirkstoff | Aufwandmenge an Wirkstoff in g/ha | % Wirkungsgrad |
| 4 | 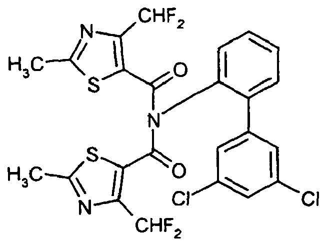 | 750 | 95 |

## Patentansprüche

1. Thiazolylbiphenylamide der Formel (I) 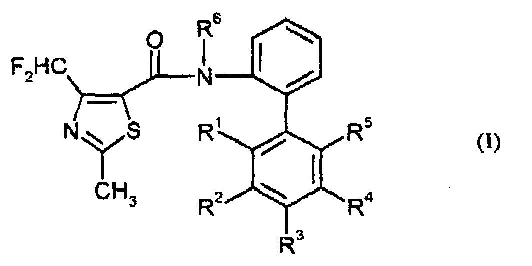 in welcher
R¹, R², R³, R⁴ und R⁵ unabhängig voneinander für Wasserstoff, Halogen, Cyano, Nitro, C₁-C₆- Alkyl, C₂-C₆-Alkenyl, C₁-C₄-Alkoxy, C₁-C₄-Alkylthio, C₁-C₄-Alkylsulfonyl, C₃-C₆- Cycloalkyl, oder für C₁-C₄-Halopenalkyl, C₁-C₄-Halogenalkoxy, C₁-C₄- Halogenalkylthio oder C₁-C₄-Halogenalkylsulfonyl mit jeweils 1 bis 5 Halogenatomen stehen,
R¹ und R² oder R² und R³ außerdem gemeinsam für gegebenenfalls durch Halogen oder C₁-C₆- Alkyl substituiertes Alkenylen stehen,
R⁶ für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylsulfanyl, C₁-C₄- Halogenalkylsulfinyl, C₁-C₄-Halogenalkylsulfonyl, Halogen-C₁-C₄-alkoxy-C₁-C₄- alkyl, C₃-C₈-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
R⁷ für Wasserstoff, C₁-C₈-Alkyl, C₁-C₈-Alkoxy, C₁-C₄-Alkoxy-C₁-C₄-alkyl, C₃-C₈- Cycloalkyl; C₁-C₆-Halogenalkyl, C₁-C₆-Halogenalkoxy, Halogen-C₁-C₄-alkoxy-C₁-C₄- alkyl, C₃-C₈-Halogencycloalkyl mit jeweils 1 bis 9 Fluor-, Chlor- und/oder Bromatomen oder 4-(Difluormethyl)2-methyl-1,3-thiazol-2-yl steht,
R⁸ und R⁹ unabhängig voneinander für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈- Halogencycloalkyl mit jeweils 1 bis 9 Fluor-, Chlor- und/oder Bromatomen stehen,
R⁸ und R⁹ außerdem gemeinsam mit dem Stickstoffatom, an das sie gebunden sind, einen ge- gebenenfalls einfach oder mehrfach, gleich oder verschieden durch Halogen oder C₁- C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
R¹⁰ und R¹¹ unabhängig voneinander für Wasserstoff, C₁-C₈-Alkyl, C₃-C₈-Cycloalkyl; C₁-C₈- Halogenalkyl, C₃-C₈-Halogencycloalkyₗ mit jeweils 1 bis 9 Fluor-, Chlor- und/oder Bromatomen stehen,
R¹⁰ und R¹¹ außerdem gemeinsam mit dem Stickstoffatom, an das sie gebunden sind, einen ge- gebenenfalls einfach oder mehrfach, gleich oder verschieden durch Halogen oder C₁- C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
R¹² für Wasserstoff oder C₁-C₆-Alkyl steht.

2. Thiazolylbiphenylamide der Formel (I) gemäß Anspruch 1, in welcher
R¹, R², R³, R⁴ und R⁵ unabhängig voneinander für Wasserstoff, Fluor, Chlor, Brom, Cyano, Nitro, Methyl, Ethyl, n- oder iso-Propyl, n-, iso-, sec- oder tert-Butyl, Methoxy, Ethoxy, Methylthio, Ethylthio, n- oder iso-Propylthio, Cyclopropyl, Trifluormethyl, Trichlormethyl, Trifluorethyl, Difluormethoxy, Trifluormethoxy, Difluorchlormethoxy, Trifluorethoxy, Difluormethylthio, Difluorchlormethylthio oder Trifluormethylthio stehen,
R¹ und R² oder R² und R³ außerdem gemeinsam für gegebenenfalls durch Fluor, Chlor, Brom oder Methyl substituiertes Butadien-diyl stehen,
R⁶ für C₁-C₆-Alkyl, C₁-C₄-Alkylsulfinyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylsulfanyl, C₁-C₄- Halogenalkylsulfinyl, C₁-C₄-Halogenalkylsulfonyl, Halogen-C₁-C₃-alkoxy-C₁-C₃- alkyl, C₃-C₆-Halogencycloalkyl mit jeweils 1 bis 9 Fluor-, Chlor- und/oder Bromatomen; -COR⁷, -CONR⁸R⁹ oder -CH₂NR¹⁰R¹¹ steht,
R⁷ für Wasserstoff, C₁-C₆-Alkyl, C₁-C₄-Alkoxy, C₁-C₃-Alkoxy-C₁-C₃-alkyl, C₃-C₆- Cycloalkyl; C₁-C₄-Halogenalkyl, C₁-C₄-Halogenalkoxy, Halogen-C₁-C₃-alkoxy-C₁-C₃- alkyl, C₃-C₆-Halogencycloalkyl mit jeweils 1 bis 9 Fluor-, Chlor- und/oder Bromatomen oder 4-(Difluormethyl)-2-methyl-1,3-thiazol-2-yl steht,
R⁸ und R⁹ unabhängig voneinander für Wasserstoff, C₁-C₆-Alkyl, C₁-C₃-Alkoxy-C₁-C₂-alkyl, C₃-C₆-Cycloalkyl; C₁-C₄-Halogenalkyl, Halogen-C₁-C₃-alkoxy-C₁-C₃-alkyl, C₃-C₆- Halogencycloalkyl mit jeweils 1 bis 9 Fluor-, Chlor- und/oder Bromatome stehen,
R⁸ und R⁹ außerdem gemeinsam mit dem Stickstoffatom, an das sie gebunden sind, einen ge- gebenenfalls einfach bis vierfach, gleich oder verschieden durch Halogen oder C₁-C₄- Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
R¹⁰ und R¹¹ unabhängig voneinander für Wasserstoff, C₁-C₆-Alkyl, C₃-C₆-Cycloalkyl; C₁-C₄- Halogenalkyl, C₃-C₆-Halogencycloalkyl mit jeweils 1 bis 9 Fluor-, Chlor- und/oder Bromatomen stehen,
R¹⁰ und R¹¹ außerdem gemeinsam mit dem Stickstoffatom, an das sie gebunden sind, bevor- zugt einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹² enthalten kann,
R¹² für Wasserstoff oder C₁-C₄-Alkyl steht.

3. Thiazolyibiphenylamide der Formel (I) gemäß Anspruch 1, in welcher
R¹, R², R³, R⁴ und R⁵ unabhängig voneinander für Wasserstoff, Fluor, Chlor, Brom, Cyano, Methyl, Methoxy, Methylthio, Trifluonnethyl, Difluormethoxy, Trifluormethoxy, Difluormethylthio oder Trifluormethylthio stehen,
R⁶ für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert- Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxyethyl, Ethoxyethyl, Cyclopropyl, Cyclopentyl, Cyclohexyl, Trifluormethyl, Trichlormethyl, Trifluorethyl, Difluormethylsulfanyl, Difluorchlormethylsulfanyl, Trifluormethylsulfanyl, Trifluor- methylsulfinyl, Trifluormethylsulfonyl, Trifluormethoxymethyl; -COR⁷, -CONR⁸R⁹ oder -CH₂NR¹⁰R¹¹ steht,
R⁷ für Wasserstoff, Methyl, Ethyl, n- oder iso-Propyl, tert-Butyl, Methoxy, Ethoxy, tert- Butoxy, Cyclopropyl; Trifluormethyl, Trifluormethoxy oder 4-(Difluormcthyl)-2- methyl-1,3-thiazol-2-yl steht,
R⁸ und R⁹ unabhängig voneinander für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl stehen,
R⁸ und R⁹ außerdem gemeinsam mit dem Stickstoffatom, an das sie gebunden sind, einen ge- gebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin bilden, wobei das Piperazin am zweiten Stickstoffatom durch R¹² substituiert sein kann,
R¹⁰ und R¹¹ unabhängig voneinander für Wasserstoff, Methyl, Ethyl, n- oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl stehen,
R¹⁰ und R¹¹ außerdem gemeinsam mit dem Stickstoffatom, an das sie gebunden sind, einen ge- gebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin bilden, wobei das Piperazin am zweiten Stickstoffatom durch R¹² substituiert sein kann,
R¹² für Wasserstoff, Methyl, Ethyl, n- oder iso-Propyl, n-, iso-, sec- oder tert-Butyl steht.

4. Thiazolylbiphenylamide der Formel (I) gemäß Anspruch 1, in welcher jeweils vier der Reste R¹, R², R³, R⁴ und R⁵ für Wasserstoff stehen.

5. Thiazolylbiphenylamide der Formel (I) gemäß Anspruch 1, in welcher
R¹, R², R⁴ und R⁵ jeweils für Wasserstoff stehen und
R³ die in einem der Ansprüche 1 bis 3 angegebenen Bedeutungen hat.

6. Thiazolylbiphenylamide der Formel (I) gemäß Anspruch 1, in welcher
R², R⁴ und R⁵ für jeweils Wasserstoff stehen und
R¹ und R³ unabhängig voneinander die in einem der Ansprüche 1 bis 3 angegebenen Bedeutungen haben.

7. Thiazolylbiphenylamide der Formel (I) gemäß Anspruch 1, in welcher
R¹, R⁴ und R⁵ jeweils für Wasserstoff stehen und
R² und R³ unabhängig voneinander die in einem der Ansprüche 1 bis 3 angegebenen Bedeutungen haben.

8. Thiazolylbiphenylamide der Formel (I) gemäß Anspruch 1, in welcher
R¹, R³ und R⁵ jeweils für Wasserstoff stehen und
R² und R⁴ unabhängig voneinander die in einem der Ansprüche 1 bis 3 angegebenen Bedeutungen haben.

9. Thiazolylbiphenylamide der Formel (I) gemäß Anspruch 1, in welcher
R⁶ für -COR⁷ steht und R⁷ für 4-(Difluormethyl)-2-methyl-1,3-thiazol-2-yl steht.

10. Thiazolylbiphenylamidc der Formel (I) gemäß Anspruch 1, in welcher
R⁶ für -COR⁷ steht und R⁷ für Methyl, Ethyl, Cyclopropyl oder Trifluormethyl, insbesondere Methyl steht.

11. Thiazolylbiphenylamide der Formel (I) gemäß Anspruch 1, in welcher
R⁶ für -CHO steht.

12. Thiazolylbiphenylamide der Formel (I) gemäß Anspruch 1, in welcher
R⁶ für Methyl, Ethyl, n- oder iso-Propyl, n-, iso-, sec- oder tert-Butyl, Methylsulfinyl, Methylsulfonyl, Methoxymethyl, Ethoxyethyl, Cyclopropyl, Cyclopentyl, Cyclohexyl, Trifluormethyl, Trichlormethyl, Trifluormethylsulfanyl, Trifluonnethylsulfinyl, Trifluormethylsulfonyl, Trifluormethoxymethyl, insbesondere Methyl, iso-Propyl oder Cyclopropyl steht.

13. Verfahren zum Herstellen der Thiazolylbiphenylamide der Formel (I) gemäß Anspruch 1, **dadurch gekennzeichnet, dass** man
(A) Thiazolylbiphenylamide der Formel (II) 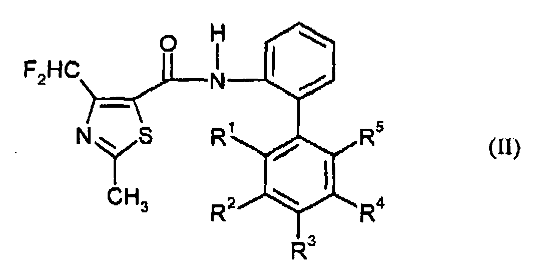 in welcher
R¹, R², R³, R⁴ und R⁵ die in Anspruch 1 angegebenen Bedeutungen haben,
mit einem Halogenid der Formel (III)
R⁶-X (III)
in welcher
R⁶ die in Anspruch 1 angegebenen Bedeutungen hat und
X für Chlor, Brom oder Iod steht,
in Gegenwart einer Base und in Gegenwart eines Verdünnungsmittels umsetzt.

14. Mittel zum Bekämpfen unerwünschter Mikroorganismen, **gekennzeichnet durch** einen Gehalt an mindestens einem Thiazolylbiphenylamid der Formel (I) gemäß Anspruch 1 neben Streckmitteln und/oder oberflächenaktiven Stoffen.

15. Verwendung von Thiazolylbiphenylamiden der Formel (I) gemäß Anspruch 1 zum Bekämpfen unerwünschter Mikroorganismen *im Pflanzenschutz und Materialschutz.*

16. Verfahren zum Bekämpfen unerwünschter Mikroorganismen *im Pflanzenschutz und Materialschutz,* **dadurch gekennzeichnet, dass** man Thiazolylbiphenylamide der Formel (I) gemäß Anspruch 1 auf die Mikroorganismen und/oder deren Lebensraum ausbringt.

17. Verfahren zum Herstellen von Mitteln zum Bekämpfen unerwünschter Mikroorganismen, **dadurch gekennzeichnet, dass** man Thiazolylbiphenylamid der Formel (I) gemäß Anspruch 1 mit Streckmitteln und/oder oberflächenaktiven Stoffen vermischt.

## Claims

1. Thiazolylbiphenylamides of the formula (I) 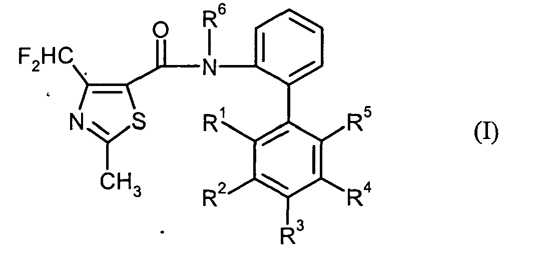 in which
R¹, R², R³, R⁴ and R⁵ independently of one another represent hydrogen, halogen, cyano, nitro, C₁-C₆-alkyl, C₂-C₆-alkenyl, C₁-C₄-alkoxy, C₁-C₄-alkylthio, C₁-C₄-alkylsulfonyl, C₃-C₆-cycloalkyl, or represent C₁-C₄-haloalkyl, C₁-C₄-haloalkoxy, C₁-C₄-haloalkylthio or C₁-C₄-haloalkylsulfonyl having in each case 1 to 5 halogen atoms,
R¹ and R² or R² and R³ furthermore together represent optionally halogen- or C₁-C₆-alkyl-substituted alkenylene,
R⁶ represents C₁-C₈-alkyl, C₁-C₆-alkylsulfinyl, C₁-C₆-alkylsulfonyl, C₁-C₄-alkoxy-C₁-C₄-alkyl, C₃-C₈-cycloalkyl; C₁-C₆-haloalkyl, C₁-C₄-haloalkylsulfanyl, C₁-C₄-haloalkylsulfinyl, C₁-C₄-haloalkyl- sulfonyl, halo-C₁-C₄-alkoxy-C₁-C₄-alkyl, C₃-C₈-halocycloalkyl having in each case 1 to 9 fluorine, chlorine and/or bromine atoms; -COR⁷, -CONR⁸R⁹ or -CH₂NR¹⁰R¹¹,
R⁷ represents hydrogen, C₁-C₈-alkyl, C₁-C₈-alkoxy, C₁-C₄-alkoxy- C₁-C₄-alkyl, C₃-C₈-cycloalkyl; C₁-C₆-haloalkyl, C₁-C₆-haloalkoxy, halo-C₁-C₄-alkoxy-C₁-C₄-alkyl, C₃-C₈-halocycloalkyl having in each case 1 to 9 fluorine, chlorine and/or bromine atoms, or 4-(difluoromethyl)-2-methyl-1,3-thiazol-2-yl,
R⁸ and R⁹ independently of one another represent hydrogen, C₁-C₈-alkyl, C₁-C₄-alkoxy-C₁-C₄-alkyl, C₃-C₈-cycloalkyl; C₁-C₈-haloalkyl, halo- C₁-C₄-alkoxy-C₁-C₄-alkyl, C₃-C₈-halocycloalkyl having in each case 1 to 9 fluorine, chlorine and/or bromine atoms,
R⁸ and R⁹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further non- adjacent heteroatoms from the group consisting of oxygen, sulfur and NR¹²,
R¹⁰ and R¹¹ independently of one another represent hydrogen, C₁-C₈-alkyl, C₃-C₈-cycloalkyl; C₁-C₈-haloalkyl, C₃-C₈-halocycloalkyl having in each case 1 to 9 fluorine, chlorine and/or bromine atoms,
R¹⁰ and R¹¹ furthermore together with the nitrogen atom to which they are attached form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further non- adjacent heteroatoms from the group consisting of oxygen, sulfur and NR¹²,
R¹² represents hydrogen or C₁-C₆-alkyl.

2. Thiazolylbiphenylamides of the formula (I) according to Claim 1 in which
R¹, R², R³, R⁴ and R⁵ independently of one another represent hydrogen, fluorine, chlorine, bromine, cyano, nitro, methyl, ethyl, n- or isopropyl, n-, iso-, sec- or tert-butyl, methoxy, ethoxy, methylthio, ethylthio, n- or isopropylthio, cyclopropyl, trifluoromethyl, trichloromethyl, trifluoroethyl, difluoromethoxy, trifluoromethoxy, difluorochloromethoxy, trifluoroethoxy, difluoromethylthio, difluorochloromethylthio or trifluoromethylthio,
R¹ and R² or R² and R³ furthermore together represent optionally fluorine-, chlorine-, bromine- or methyl-substituted butadienediyl,
R⁶ represents C₁-C₆-alkyl, C₁-C₄-alkylsulfinyl, C₁-C₄-alkylsulfonyl, C₁-C₃-alkoxy-C₁-C₃-alkyl, C₃-C₆-cycloalkyl; C₁-C₄-haloalkyl, C₁-C₄-haloalkylsulfanyl, C₁-C₄-haloalkylsulfinyl, C₁-C₄-haloalkyl- sulfonyl, halo-C₁-C₃-alkoxy-C₁-C₃-alkyl, C₃-C₆-halocycloalkyl having in each case 1 to 9 fluorine, chlorine and/or bromine atoms; -COR⁷, -CONR⁸R⁹ or -CH₂NR¹⁰R¹¹,
R⁷ represents hydrogen, C₁-C₆-alkyl, C₁-C₄-alkoxy, C₁-C₃-alkoxy-C₁- C₃-alkyl, C₃-C₆-cycloalkyl; C₁-C₄-haloalkyl, C₁-C₄-haloalkoxy, halo-C₁-C₃-alkoxy-C₁-C₃-alkyl, C₃-C₆-halocycloalkyl having in each case 1 to 9 fluorine, chlorine and/or bromine atoms or 4- (difluoromethyl)-2-methyl-1,3-thiazol-2-yl,
R⁸ and R⁹ independently of one another represent hydrogen, C₁-C₆-alkyl, C₁-C₃-alkoxy-C₁-C₃-alkyl, C₃-C₆-cycloalkyl; C₁-C₄-haloalkyl, halo- C₁-C₃-alkoxy-C₁-C₃-alkyl, C₃-C₆-halocycloalkyl having in each case 1 to 9 fluorine, chlorine and/or bromine atoms,
R⁸ and R⁹ furthermore together with the nitrogen atom to which they are attached form a saturated heterocycle which is optionally mono- to tetrasubstituted by identical or different substituents from the group consisting of halogen and C₁-C₄-alkyl and which has 5 to 8 ring atoms, where the heterocycle may contain 1 or 2 further non- adjacent heteroatoms from the group consisting of oxygen, sulfur and NR¹²,
R¹⁰ and R¹¹ independently of one another represent hydrogen, C₁-C₆-alkyl, C₃-C₆-cycloalkyl; C₁-C₄-haloalkyl, C₃-C₆-halocycloalkyl having in each case 1 to 9 fluorine, chlorine and/or bromine atoms,
R¹⁰ and R¹¹ furthermore together with the nitrogen atom to which they are attached preferably form a saturated heterocycle which is optionally mono- or polysubstituted by identical or different substituents from the group consisting of halogen and C₁-C₄-alkyl and which has 5 to 8 ring atoms, where the heterocycle may contain 1 or 2 further non- adjacent heteroatoms from the group consisting of oxygen, sulfur and NR¹²,
R¹² represents hydrogen or C₁-C₄-alkyl.

3. Thiazolylbiphenylamides of the formula (I) according to Claim 1 in which
R¹, R², R³, R⁴ and R⁵ independently of one another represent hydrogen, fluorine, chlorine, bromine, cyano, methyl, methoxy, methylthio, trifluoromethyl, difluoromethoxy, trifluoromethoxy, difluoromethylthio or trifluoromethylthio,
R⁶ represents methyl, ethyl, n- or isopropyl, n-, iso-, sec- or tert-butyl, pentyl or hexyl, methylsulfinyl, ethylsulfinyl, n- or isopropylsulfinyl, n-, iso-, sec- or tert-butylsulfinyl, methylsulfonyl, ethylsulfonyl, n- or isopropylsulfonyl, n-, iso-, sec- or tert- butylsulfonyl, methoxymethyl, methoxyethyl, ethoxymethyl, ethoxyethyl, cyclopropyl, cyclopentyl, cyclohexyl, trifluoromethyl, trichloromethyl, trifluoroethyl, difluoromethylsulfanyl, difluorochloromethylsulfanyl, trifluoromethylsulfanyl, trifluoromethylsulfinyl, trifluoromethylsulfonyl, tri- fluoromethoxymethyl; -COR⁷, -CONR⁸R⁹ or -CH₂NR¹⁰R¹¹,
R⁷ represents hydrogen, methyl, ethyl, n- or isopropyl, tert-butyl, methoxy, ethoxy, tert-butoxy, cyclopropyl; trifluoromethyl, trifluoromethoxy or 4-(difluoromethyl)-2-methyl-1,3-thiazol-2-yl,
R⁸ and R⁹ independently of one another represent hydrogen, methyl, ethyl, n- or isopropyl, n-, iso-, sec- or tert-butyl, methoxymethyl, methoxyethyl, ethoxymethyl, ethoxyethyl, cyclopropyl, cyclopentyl, cyclohexyl; trifluoromethyl, trichloromethyl, trifluoroethyl, trifluoromethoxymethyl,
R⁸ and R⁹ furthermore together with the nitrogen atom to which they are attached form a saturated heterocycle from the group consisting of morpholine, thiomorpholine and piperazine, which heterocycle is optionally mono- to tetrasubstituted by identical or different substituents from the group consisting of fluorine, chlorine, bromine and methyl, where the piperazine may be substituted on the second nitrogen atom by R¹²,
R¹⁰ and R¹¹ independently of one another represent hydrogen, methyl, ethyl, n- or isopropyl, n-, iso-, sec- or tert-butyl, methoxymethyl, methoxyethyl, ethoxymethyl, ethoxyethyl, cyclopropyl, cyclopentyl, cyclohexyl; trifluoromethyl, trichloromethyl, trifluoroethyl, trifluoromethoxymethyl,
R¹⁰ and R¹¹ furthermore together with the nitrogen atom to which they are attached form a saturated heterocycle from the group consisting of morpholine, thiomorpholine and piperazine, which heterocycle is optionally mono- to tetrasubstituted by identical or different substituents from the group consisting of fluorine, chlorine, bromine and methyl, where the piperazine may be substituted on the second nitrogen atom by R¹²,
R¹² represents hydrogen, methyl, ethyl, n- or isopropyl, n-, iso-, sec- or tert-butyl.

4. Thiazolylbiphenylamides of the formula (I) according to Claim 1 in which in each case four of the radicals R¹, R², R³, R⁴ and R⁵ represent hydrogen.

5. Thiazolylbiphenylamides of the formula (I) according to Claim 1 in which
R¹, R², R⁴ and R⁵ each represent hydrogen and
R³ is as defined in any of Claims 1 to 3.

6. Thiazolylbiphenylamides of the formula (I) according to Claim 1 in which
R², R⁴ and R⁵ each represent hydrogen and
R¹ and R³ independently of one another are as defined in any of Claims 1 to 3.

7. Thiazolylbiphenylamides of the formula (I) according to Claim 1 in which
R¹, R⁴ and R⁵ each represent hydrogen and
R² and R³ independently of one another are as defined in any of Claims 1 to3.

8. Thiazolylbiphenylamides of the formula (I) according to Claim 1 in which
R¹, R³ and R⁵ each represent hydrogen and
R² and R⁴ independently of one another are as defined in any of Claims 1 to3.

9. Thiazolylbiphenylamides of the formula (I) according to Claim 1 in which
R⁶ represents -COR⁷ and R⁷ represents 4-(difluoromethyl)-2-methyl- 1,3-thiazol-2-yl.

10. Thiazolylbiphenylamides of the formula (I) according to Claim 1 in which
R⁶ represents -COR⁷ and R⁷ represents methyl, ethyl, cyclopropyl or trifluoromethyl, in particular methyl.

11. Thiazolylbiphenylamides of the formula (I) according to Claim 1 in which
R⁶ represents -CHO.

12. Thiazolylbiphenylamides of the formula (I) according to Claim 1 in which
R⁶ represents methyl, ethyl, n- or isopropyl, n-, iso-, sec- or tert-butyl, methylsulfinyl, methylsulfonyl, methoxymethyl, ethoxyethyl, cyclo- propyl, cyclopentyl, cyclohexyl, trifluoromethyl, trichloromethyl, trifluoromethylsulfanyl, trifluoromethylsulfinyl, trifluoromethylsulfonyl, trifluoromethoxymethyl, in particular methyl, isopropyl or cyclopropyl.

13. Process for preparing the thiazolylbiphenylamides of the formula (I) according to Claim 1, **characterized in that**
(A) thiazolylbiphenylamides of the formula (II) 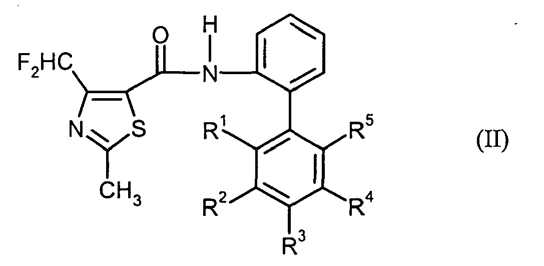 in which
R¹, R², R³, R⁴ and R⁵ are as defined in Claim 1
are reacted with a halide of the formula (III)
R⁶ ―X (III)
in which
R⁶ is as defined in Claim 1 and
X represents chlorine, bromine or iodine
in the presence of a base and in the presence of a diluent.

14. Composition for controlling unwanted microorganisms, **characterized in that** it comprises at least one thiazolylbiphenylamides of the formula (I) according to Claim 1, in addition to extenders and/or surfactants.

15. Use of thiazolylbiphenylamides of the formula (I) according to Claim 1 for controlling unwanted microorganisms in crop protection and in the protection of materials.

16. Method of controlling unwanted microorganisms in crop protection and in the protection of materials, **characterized in that** thiazolylbiphenylamides of the formula (I) according to Claim 1 are applied to the microorganisms and/or their habitat.

17. Process for preparing compositions for controlling unwanted microorganisms, **characterized in that** thiazolylbiphenylamides of the formula (I) according to Claim 1 is mixed with extenders and/or surfactants.

## Revendications

1. Thiazolylbiphénylamides de formule (I) 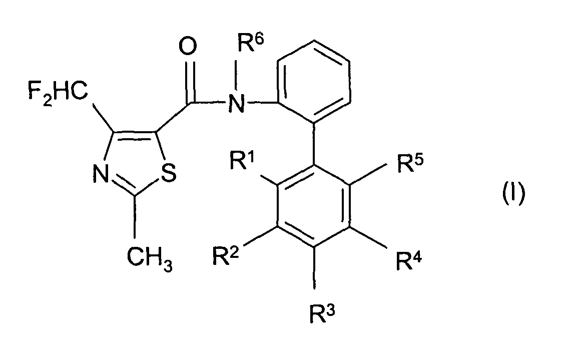 où
R¹, R², R³, R⁴ et R⁵ représentent indépendamment les uns des autres l'hydrogène, un halogène, cyano nitro, C₁-C₆-alkyle, C₂-C₆-alcényle, C₁-C₄-alcoxy, C₁-C₄-alkylthio, C₁-C₄-alkylsulfonyle, C₃-C₆-cydoalkyle, ou C₁-C₄-halogénoalkyle, C₁-C₄-halogénoalcoxy, C₁-C₄- halogénoalkylthio ou C₁-C₄-halogénoalkylsulfonyle ayant dans chaque cas 1 à 5 atomes d'halogène,
R¹ et R² ou R² et R³ représentent en outre conjointement alcénylène éventuellement substitué par halogène ou C₁-C₆-alkyle,
R⁶ représente C₁-C₈-alkyle, C₁-C₆-alkylsulfinyle, C₁-C₆-alkylsulfonyle, C₁-C₄-alcoxy-C₁-C₄-alkyle, C₃-C₈-cycloalkyle ; C₁-C₆-halogénoalkyle, C₁-C₄-halogénoalkylsulfanyle, C₁-C₄-halogénoalkylsulfinyle, C₁-C₄- halogénoalkylsulfonyle, halogéno-C₁-C₄-alcoxy-C₁-C₄-alkyle, C₃-C₈- halogénocycloalkyle ayant dans chaque cas 1 à 9 atomes de fluor, de chlore et/ou de brome ; -COR⁷, -CONR⁸R⁹ ou -H₂NR¹⁰R¹¹,
R⁷ représente l'hydrogène, C₁-C₈-alkyle, C₁-C₈-alcoxy, C₁-C₄-alcoxy- C₁-C₄-alkyle, C₃-C₈-cycloalkyle ; C₁-C₆-haogénoalkyle, C₁-C₆- halogénoalcoxy, halogéno-C₁-C₄-alcoxy-C₁-C₄-alkyle, C₃-C₈- halogénocycloalkyle ayant dans chaque cas 1 à 9 atomes de fluor, de chlore et/ou de brome ou 4-(difluorométhyl)-2-méthyl-1,3- thiazol-2-yle,
R⁸ et R⁹ représentent indépendamment l'un de l'autre l'hydrogène, C₁-C₈- alkyle, C₁-C₄-alcoxy-C₁-C₄-alkyle, C₃-C₈-cycloalkyle ; C₁-C₈-halogéno- alkyle, halogéno-C₁-C₄-alcoxy-C₁-C₄-alkyle, C₃-C₈-halogénocycloalkyle ayant dans chaque cas 1 à 9 atomes de fluor, de chlore et/ou de brome,
R⁸ et R⁹ forment en outre avec l'atome d'azote auquel ils sont liés un hétérocycle à 5 à 8 atomes cycliques saturé éventuellement substitué une ou plusieurs fois, de manière identique ou différente, par halogène ou C₁-C₄-alkyle, où l'hétérocycle peut contenir 1 ou 2 autres hétéroatomes non voisins de la série de l'oxygène, du soufre ou NR¹²,
R¹⁰ et R¹¹ représentent indépendamment l'un de l'autre l'hydrogène, C₁- C₈-alkyle, C₃-C₈-cycloalkyle ; C₁-C₈-halogénoalkyle, C₃-C₈-halogéno- cycloalkyle ayant dans chaque cas 1 à 9 atomes de fluor, de chlore et/ou de brome,
R¹⁰ et R¹¹ forment en outre avec l'atome d'azote auquel ils sont liés un hétérocycle à 5 à 8 atomes cycliques saturé éventuellement substitué une ou plusieurs fois, de manière identique ou différente, par halogène ou C₁-C₄-alkyle, où l'hétérocycle peut contenir 1 ou 2 autres hétéroatomes non voisins de la série de l'oxygène, du soufre ou NR¹²,
R¹² représente l'hydrogène ou C₁-C₆-alkyle.

2. Thiazolylbiphénylamides de formule (I) selon la revendication 1 où
R¹, R², R³, R⁴ et R⁵ représentent indépendamment les uns des autres l'hydrogène, le fluor, le chlore, le brome, cyano, nitro, méthyle, éthyle, n- ou iso-propyle, n-, iso-, sec- ou tert-butyle, méthoxy, éthoxy, méthylthio, éthylthio, n- ou iso-propylthio, cyclopropyle, trifluorométhyle, trichlorométhyle, trifluoroéthyle, difluorométhoxy, trifluorométhoxy, difluorochlorométhoxy, trifluoroéthoxy, difluorométhylthio, difluorochlorométhylthio ou trifluorométhylthio,
R¹ et R² ou R² et R³ représentent en outre conjointement butadiènediyle éventuellement substitué par le fluor, le chlore, le brome ou méthyle,
R⁶ représente C₁-C₆-alkyle, C₁-C₄-alkylsulfinyle, C₁-C₄-alkylsulfonyle, C₁-C₃-alcoxy-C₁-C₃-alkyle, C₃-C₆-cycloalkyle ; C₁-C₄-halogénoalkyle, C₁-C₄-halogénoalkylsulfanyle, C₁-C₄-halogénoalkylsulfinyle, C₁-C₄- halogénoalkylsulfonyle, halogéno-C₁-C₃-alcoxy-C₁-C₃-alkyle, C₃-C₆- halogénocycloalkyle ayant dans chaque cas 1 à 9 atomes de fluor, de chlore et/ou de brome ; -COR⁷, -CONR⁸R⁹ ou -CH₂NR¹⁰R¹¹,
R⁷ représente l'hydrogène, C₁-C₆-alkyle, C₁-C₄-alcoxy, C₁-C₃-alcoxy-C₁- C₃-alkyle, C₃-C₆-cycloalkyle ; C₁-C₄-halogénoalkyle, C₁-C₄-halogéno- alcoxy, halogéno-C₁-C₃-alcoxy-C₁-C₃-alkyle, C₃-C₆-halogénocyclo- alkyle ayant dans chaque cas 1 à 9 atomes de fluor, de chlore et/ou de brome ou 4-(difluorométhyl)-2-méthyl-1,3-thiazol-2-yle,
R⁸ et R⁹ représentent indépendamment l'un de l'autre l'hydrogène, C₁-C₆- alkyle, C₁-C₃-alcoxy-C₁-C₃-alkyle, C₃-C₆-cycloalkyle ; C₁-C₄-halogéno- alkyle, halogéno-C₁-C₃-alcoxy-C₁-C₃-alkyle, C₃-C₆-halogénocycloalkyle ayant dans chaque cas 1 à 9 atomes de fluor, de chlore et/ou de brome,
R⁸ et R⁹ forment en outre avec l'atome d'azote auquel ils sont liés un hétérocycle à 5 à 8 atomes cycliques saturé éventuellement substitué 1 à 4 fois, de manière identique ou différente, par halogène ou C₁-C₄-alkyle, où l'hétérocycle peut contenir 1 ou 2 autres hétéroatomes non voisins de la série de l'oxygène, du soufre ou NR¹²,
R¹⁰ et R¹¹ représentent indépendamment l'un de l'autre l'hydrogène, C₁- C₆-alkyle, C₃-C₆-cycloalkyle ; C₁-C₄-halogénoalkyle, C₃-C₆-halogéno- cycloalkyle ayant dans chaque cas 1 à 9 atomes de fluor, de chlore et/ou de brome,
R¹⁰ et R¹¹ forment en outre avec l'atome d'azote auquel ils sont liés de préférence un hétérocycle à 5 à 8 atomes cycliques saturé éventuellement substitué une ou plusieurs fois, de manière identique ou différente, par halogène ou C₁-C₄-alkyle, où l'hétérocycle peut contenir 1 ou 2 autres hétéroatomes non voisins de la série de l'oxygène, du soufre ou NR¹²,
R¹² représente l'hydrogène ou C₁-C₄-alkyle.

3. Thiazolylbiphénylamides de formule (I) selon la revendication 1 où
R¹, R², R³, R⁴ et R⁵ représentent indépendamment les uns des autres l'hydrogène, le fluor, le chlore, le brome, cyano, méthyle, méthoxy, méthylthio, trifluorométhyle, difluorométhoxy, trifluorométhoxy, difluorométhylthio ou trifluorométhylthio,
R⁶ représente méthyle, éthyle, n- ou iso-propyle, n-, iso-, sec- ou tert- butyle, pentyle ou hexyle, méthylsulfinyle, éthylsulfinyle, n- ou iso- propylsulfinyle, n-, iso-, sec- ou tert-butylsulfinyle, méthylsulfonyle, éthylsulfonyle, n- ou iso-propylsulfonyle, n-, iso-, sec- ou tert-butyl- sulfonyle, méthoxyméthyle, méthoxyéthyle, éthoxyméthyle, éthoxyéthyle, cyclopropyle, cyclopentyle, cyclohexyle, trifluorométhyle, trichlorométhyle, trifluoroéthyle, difluorométhyl- sulfanyle, difluorochlorométhylsulfanyle, trifluorométhylsulfanyle, trifluorométhylsulfinyle, trifluorométhylsulfonyle, trifluorométhoxy- méthyle ; -COR⁷, -CONR⁸R⁹ ou -CH₂NR¹⁰R¹¹,
R⁷ représente l'hydrogène, méthyle, éthyle, n- ou iso-propyle, tert- butyle, méthoxy, éthoxy, tert-butoxy, cyclopropyle ; trifluorométhyle, trifluorométhoxy ou 4-(difluorométhyl)-2-méthyl-1,3-thiazol-2-yle,
R⁸ et R⁹ représentent indépendamment l'un de l'autre l'hydrogène, méthyle, éthyle, n- ou iso-propyle, n-, iso-, sec- ou tert-butyle, méthoxyméthyle, méthoxyéthyle, éthoxyméthyle, éthoxyéthyle, cyclopropyle, cyclopentyle, cyclohexyle ; trifluorométhyle, trichlorométhyle, trifluoroéthyle, trifluorométhoxyméthyle,
R⁸ et R⁹ forment en outre avec l'atome d'azote auquel ils sont liés un hétérocycle saturé éventuellement substitué 1 à 4 fois, de manière identique ou différente, par le fluor, le chlore, le brome ou méthyle de la série de la morpholine, de la thiomorpholine ou de la pipérazine, où la pipérazine peut être substituée par R¹² sur le deuxième atome d'azote,
R¹⁰ et R¹¹ représentent indépendamment l'un de l'autre l'hydrogène, méthyle, éthyle, n- ou iso-propyle, n-, iso-, sec- ou tert-butyle, méthoxyméthyle, méthoxyéthyle, éthoxyméthyle, éthoxyéthyle, cyclopropyle, cyclopentyle, cyclohexyle ; trifluorométhyle, trichlorométhyle, trifluoroéthyle, trifluorométhoxyméthyle,
R¹⁰ et R¹¹ forment en outre avec l'atome d'azote auquel ils sont liés un hétérocycle saturé éventuellement substitué 1 à 4 fois, de manière identique ou différente, par le fluor, le chlore, le brome ou méthyle, de la série de la morpholine, de la thiomorpholine ou de la pipérazine, où la pipérazine peut être substitué par R¹² sur le deuxième atome d'azote,
R¹² représente l'hydrogène, méthyle, éthyle, n- ou iso-propyle, n-, iso-, sec- ou tert-butyle.

4. Thiazolylbiphénylamides de formule (I) selon la revendication 1 où, dans chaque cas, quatre des groupements R¹, R², R³, R⁴ et R⁵ représentent l'hydrogène.

5. Thiazolylbiphénylamides de formule (I) selon la revendication 1, où
R¹, R², R⁴ et R⁵ représentent chacun l'hydrogène et
R³ a les significations indiquées dans l'une des revendications 1 à 3.

6. Thiazolylbiphénylamides de formule (I) selon la revendication 1, où
R², R⁴ et R⁵ représentent chacun l'hydrogène et
R¹ et R³ ont indépendamment l'un de l'autre les significations indiquées dans l'une des revendications 1 à 3.

7. Thiazolylbiphénylamides de formule (I) selon la revendication 1, où
R¹, R⁴ et R⁵ représentent chacun l'hydrogène et
R² et R³ ont indépendamment l'un de l'autre les significations indiquées dans l'une des revendications 1 à 3.

8. Thiazolylbiphénylamides de formule (I) selon la revendication 1, où
R¹, R³ et R⁵ représentent chacun l'hydrogène et
R² et R⁴ ont indépendamment l'un de l'autre les significations indiquées dans l'une des revendications 1 à 3.

9. Thiazolylbiphénylamides de formule (I) selon la revendication 1, où
R⁶ représente -COR⁷ et R⁷ représente 4-(difluorométhyl)-2-méthyl-1,3- thiazol-2-yle.

10. Thiazolylbiphénylamides de formule (I) selon la revendication 1, où
R⁶ représente -COR⁷ et R⁷ représente méthyle, éthyle, cyclopropyle ou trifluorométhyle, en particulier méthyle.

11. Thiazolylbiphénylamides de formule (I) selon la revendication 1, où
R⁶ représente -CHO.

12. Thiazolylbiphénylamides de formule (I) selon la revendication 1, où
R⁶ représente méthyle, éthyle, n- ou iso-propyle, n-, iso-, sec- ou tert- butyle, méthylsulfinyle, méthylsulfonyle, méthoxyméthyle, éthoxy- éthyle, cyclopropyle, cyclopentyle, cyclohexyle, trifluorométhyle, trichlorométhyle, trifluorométhylsulfanyle, trifuorométhylsulfinyle, trifluorométhylsulfonyle, trifluorométhoxyméthyle, en particulier méthyle, iso-propyle ou cyclopropyle.

13. Procédé de production des thiazolylbiphénylamides de formule (I) selon la revendication 1, **caractérisé en ce que** l'on fait réagir
(A) des thiazolylbiphénylamides de formule (II) 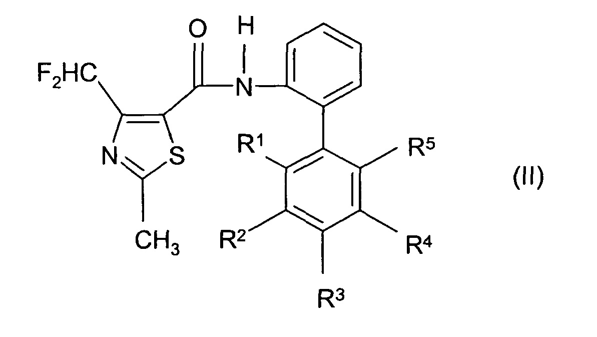 où
R¹, R², R³, R⁴ et R⁵ ont les significations indiquées dans la revendi cation 1
avec un halogénure de formule (III)
R⁶-X (III)
où
R⁶ a les significations indiquées dans la revendication 1 et
X représente le chlore, le brome ou l'iode,
en présence d'une base et en présence d'un diluant.

14. Agent pour la lutte contre les micro-organismes indésirables, **caractérisé par** une teneur en au moins un thiazolylbiphénylamide de formule (I) selon la revendication 1, outre des diluants et/ou des substances tensioactives.

15. Utilisation de thiazolylbiphénylamides de formule (I) selon la revendication 1 pour la lutte contre les micro-organismes indésirables dans la protection des plantes et la protection des matériaux.

16. Procédé pour la lutte contre les micro-organismes indésirables dans la protection des plantes et la protection des matériaux, **caractérisé en ce que** l'on applique des thiazolylbiphénylamides de formule (I) selon la revendication 1 sur les micro-organismes et/ou leur habitat.

17. Procédé de production d'agents pour la lutte contre les micro-organismes indésirables, **caractérisé en ce que** l'on mélange un thiazolylbiphénylamide de formule (I) selon la revendication 1 avec des diluants et/ou des substances tensioactives.
